Europäisches Patentamt

European Patent Office

Office européen des brevets

(19)

(11) Veröffentlichungsnummer: **0 141 022**
**B1**

(12) **EUROPÄISCHE PATENTSCHRIFT**

(45) Veröffentlichungstag der Patentschrift: **13.06.90**

(51) Int. Cl.⁵: **A 61 F 2/30,** A 61 F 2/32

(21) Anmeldenummer: **84106967.7**

(22) Anmeldetag: **18.06.84**

(54) **Verankerungsschaft für eine Endoprothese.**

(30) Priorität: **30.08.83 DE 3331163**

(43) Veröffentlichungstag der Anmeldung:
**15.05.85 Patentblatt 85/20**

(45) Bekanntmachung des Hinweises auf die
Patenterteilung:
**13.06.90 Patentblatt 90/24**

(84) Benannte Vertragsstaaten:
**AT BE CH DE FR GB IT LI NL SE**

(56) Entgegenhaltungen:
**DE-A-32 165 39**
**DE-B-2 517 702**
**DE-B-2 834 155**

(73) Patentinhaber: **GEBRÜDER SULZER
AKTIENGESELLSCHAFT
Zürcherstrasse 9
CH-8401 Winterthur (CH)**

(72) Erfinder: **Frey, Otto
Wallrütistrasse 56
CH-8400 Winterthur (CH)**

(74) Vertreter: **Sparing Röhl Henseler Patentanwälte
European Patent Attorneys
Rethelstrasse 123
D-4000 Düsseldorf 1 (DE)**

Courier Press, Leamington Spa, England.

**Beschreibung**

Die Erfindung betrifft einen Verankerungsschaft für eine Endoprothese, der in seinem proximalen Bereich mindestens auf Teilen seiner Oberfläche mit einer für das Einwachsen von Knochengewebe vorgesehenen Oberflächenstruktur aus sich abwechselnden Rippen und Tälern trägt, die in Richtung der Längsachse des Verankerungsschaftes und gegen seine Längsachse von distal nach proximal in einem spitzen Neigungswinkel verlaufen. Verankerungsschäfte der genannten Art sind z.B. aus der DE—OS 23 56 464 bekannt.

Um den vertikalen Abstand, d.h. den vertikalen Hebelarm, zwischen dem "Drehpunkt" einer Biegebelastung und dem Anssatzpunkt dieser Belastung, d.h. beispielsweise dem Gelenkkopf bei einer Hüftgelenksprothese, zu verringern, ist es mechanisch sinnvoll, die die Belastungen auf den Knochen übertragende Verankerung möglichst nahe dem proximalen Ende des Verakerungsschaftes vorzunehmen und nicht den "Drehpunkt"' und den Bereich der Weiterleitung der wesentlichen Kräfte — wie bei heute üblichen Hüftgelenksprothesen und beispielsweise auch der vorstehend erwähnten Konstruktion — in die untere, also die distale Hälfte des Schaftes zu verlegen.

In der früheren europäischen Patentanmeldung 83 104071.2 (Veröffentlichungsnummer 0 093 378) wird eine Schaftkonstruktion vorgeschlagen, bei der der sich im proximalen Bereich praktisch nicht erweiternde Schaft in diesem Bereich mit Rippen versehen ist: diese Rippen "wachsen" unter einem Winkel aus der Schaftoberfläche heraus. Bei diesem Schaft, der für eine zementfreie Verankerung bestimmt ist, erfolgt die Verankerung primär wie bisher üblich, ebenfalls im distalen Schaftbereich; denn der Knochen wird bei der Implantation dieses Schaftes bis auf die Kortikalis ausgeräumt. Die Rippen, die zur Auflage auf der Kortikalis bestimmt sind, dienen als Rotationssicherung und — nach dem Einwachsen von Gewebe in ihre Zwischenräume — zur Sekundärfixierung.

Aus dem erwähnten mechanischen Gründen ist man in letzter Zeit dazu übergegangen, beispielsweise die Verankerungsschäfte für Femurkopfprothesen im intertrochantären Bereich zu fixieren. Werden derartige Schäfte implantiert, ohne dass ihre Verankerung und Fixierung über ein Knochenzementbett erfolgen, so ist bekanntlich eines der wesentlichen Probleme die Primär-Stabilität des Schaftes im Knochen, d.h. die Fixierung des Schaftes in der ersten Zeit nach der Implantation, ehe Gewebe an den Schaft herangewachsen oder bei entsprechender Ausbildung seiner Oberfläche, siehe z.B. die DE—OS 29 14 513, in ihn eingewachsen ist. Die genannte DE—OS betrifft die Ausgestaltung und Bemessung einer Oberflächenstruktur für Implantate, durch die das Ein- und Anwachsen von Gewebe besonders gefördert wird. Sie gibt jedoch keine Anregungen für die Gewährleistung einer ausreichenden Primär-Stabilität.

Eine wirkungsvolle Massnahme zur Erzeugung der Primär-Stabilität besteht darin, den Schaft, wie an sich bekannt, mindestens im proximalen bzw. intertrochantären Bereich von distal nach proximal konisch erweitert zu gestalten, um dem Schaft in einem verdichteten Bett aus spongiösem Gewebe zu lagern und zu fixieren. Diese konische Erweiterung muss in einem relativ engen Winkelbereich erfolgen. Dabei ist einerseits die untere Grenze durch die für die Primär-Stabilität notwendige Minimalverdichtung der Spongiosa gegeben, während die obere Grenze des Winkels der konischen Erweiterung dadurch bestimmt ist, dass die von dem Konus ausgeübten radialen Kräfte nicht die Gefahr einer Sprengung des kortikalen Knochens im Umfangsrichtung auslösen dürfen. Bei geringem Oeffnungswinkel der konischen Erweiterung ist ferner die Führung des Schaftes, die am kortikalen Rand der Resektionsöffnung erfolgt, während des Einschlagens relativ gering und daher unzureichend.

Aufgabe der Erfindung ist es, eine möglichst grosse Verdichtung der Spongiosa beim Einschlagen der Prothese zu erreichen, ohne eine Sprengwirkung in der Kortikalis zu erzeugen, darüberhinaus soll bei dem neuen Schaft das Einschlagen durch eine verbesserte Führung am kortikalen Rand der Operationsöffnung erleichtert werden. Diese Aufgabe wird erfindungsgemäss dadurch gelöst, dass der Neigungswinkel α der zwischen den Rippen gelegenen Talsohlen gegen die Längsachse $1° \leq \alpha \leq 10°$, vorzugsweise $2° \leq \alpha \leq 5°$, und der Neigungswinkel β der Rippenkämme grösser als der zugehörige Neigungswinkel ist, wobei der Neigungswinkel β gleich $2° \leq \beta \leq 12°$, vorzugsweise $4° \leq \beta \leq 8°$.

Bei der neuen Construction wird der Knochen lediglich auf die Abmessungen des Schaftkernes im Scheitelbereich des Winkels α ausgeräumt, so dass beim Einschlagen eine "Basis"-Verdichtung der Spongiosa bzw. des Knochenzementes erfolgt; der Winkel α ist der halbe Konuswinkel der Erweiterung, der auch der Neigungswinkel der Talsohle gegen die Längsachse des Schaftes ist. Die Rippenkämme, die unter dem Winkel β verlaufen, dringen beim Einschlagen der Prothese tiefer in das spongiose Knochengewebe bze. in ein Knochenzementbett ein, verdrängen und verdichten Gewebe oder Knochenzement also stärker. Als Ganzes gesehen ergibt sich eine "Mischverdichtung", die bei gegebenen Winkeln α und β-die im wesentlichen durch Form und Abmessungen sowie die Verdichtungsmöglichkeit der Spongiosa des individuellen Femurknochens bestimmt sind — eine gute Primär-Stabilität ohne die Gefahr der Knochensprengung zur Folge hat; der relativ grosse Winkel β bewirkt darüberhinaus, dass der Schaft beim Implantieren relativ frühzeitig am kortikalen Rand der Resektionsöffnung geführt wird.

Der Winkel β wird dabei so gewählt, dass die Rippenkämme — von örtlichen Umgleichmässigkeiten abgesehen — nicht direkt auf der Kortikalis des Knochens aufliegen, sondern noch in spongiöses Gewebe eingebettet sind; denn der kleine E-Modul der Spongiosa wirk — wie Berechnungen anschliessende Versuche ergeben hagen — als Dämpfung und Abfederung der Auflage. Es treten daher an der Kortikalis keine ausgesprochenen Spannungsspitzen auf, sondern es findet dadurch eine kontinuierliche Kraftweiterleitung vom Schaft auf den Knochen statt.

Bei rand- oder kragenlosen Schäften erweist sich der grössere Neigungswinkel β darüberhinaus als vorteilhaft für ein "Setzen" der Prothese, durch das der Schaft im Laufe der Zeit tiefer in den Knochen eindringt, d.h. sich an das auf ihn reagierende, lebende Gewebe anpasst. Weiterhin kann mit relativ grossen Winkeln β die "Einsinktiefe" begrenzt werden; diese Begrenzung ist notwendig, damit die Lage des Gelenkkopfes relativ zum Skelett des Patienten sich nicht unzulässig weit nach unten verschiebt.

Um Hinterschneidungen, die schon bei geringen Relativbewegungen zwischen Implantat und Knochen Zugbeanspruchungen an dem zwischen die Rippen eingewachsenen Knochengewebe hervorrufen, zu vermeiden, ist es vorteilhaft, wenn die Rippenkämme und die Talsohlen ein ovales, kreis- oder ellipsenförmiges Querschnittssprofil haben, wenn ferner für den Oeffnungswinkel γ des abgerundeten Profilquerschnittsektors gilt (2/3). $\pi \leq \gamma \leq \pi$, und wenn schliesslich der zur Schaftoberfläche parallele Abstand zweier benachbarter Krümmungsmittelpunkte eines Talsohlen- und eines Kammprofils mindestens gleich der Summe der Krümmungsradien der beiden Profile multipliziert mit cos (90—γ/2) ist.

Für die Krümmungsradien der beiden Profile von Rippenkämmen und Talsohlen haben sich Werte zwischen 1 und 6 mm bewährt. Eine zusätzliche Verdichtung der Spongiosa lässt sich erreichen, wenn — in bekannter Weise — zum distalen Ende hin der Krümmungsradius für die Talsohlen zu- und derjenige für die Rippenkämme abnimmt.

Im golgenden wird die Erfindung anhand von Ausführungsbeispielen im Zusammenhang mit der Zeichnung näher erläutert.

Fig. 1 gibt in schematischer Darstellung eine seitliche Ansicht, die bei der eingesetzten Prothese einer Blickrichtung anterior/posterior entspricht, einer in einem Femurknochen eingesetzten Hüftgelenkprothese wieder;

Fig. 2 ist eine Ansicht von Fig. 1 von links;

Fig. 3 und 4 zeigen — in den Schnitten VI—VI und VII—VII von Fig. 1 entsprechenden Darstellungen — zwei Profilquerschnitte für die neue Oberflächenstruktur;

Fig. 5 und 6 schliesslich sind die Schnitte VI—VI nze. VII—VII von Fig. 1 in einem vergrösserten Massstab.

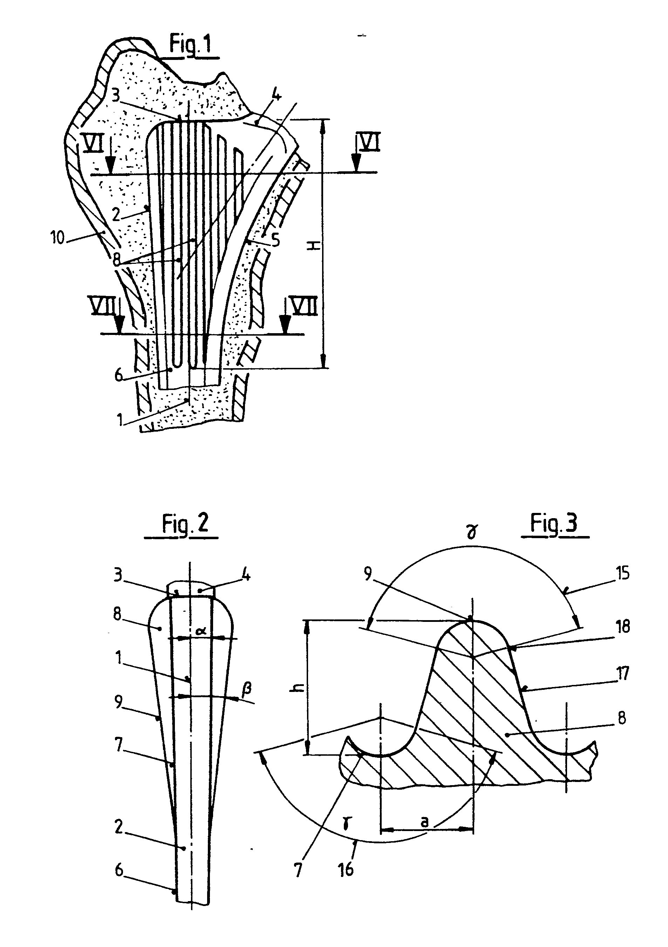

Bei der Schaftkonstruktion nach der Fig. 1 handelt es sich um einen sogenannten Geradschaft, der sich von seinem distalen Ende aus symmetrisch zu seiner — in Längsrichtung in den Femurknochen 10 einzusetzenden — Längsachse 1 (Fig. 1) konisch erweitert, wobei seine laterale Schmalseite 2 in einer mindestens nahezu horizontalen Schulter 3 endet, die den Uebergang zu einem nur im Ansatz gezeigten Prothesenhals 4 bildet.

Der sich erweiternde Konus der medialen Schmalseite 5 geht — bereits in einem Bereich distal von dem gezeigten proximalen Schaftbereich — in einen Kreisbogen über, der ebenfalls im Prothesenhals 4 endet.

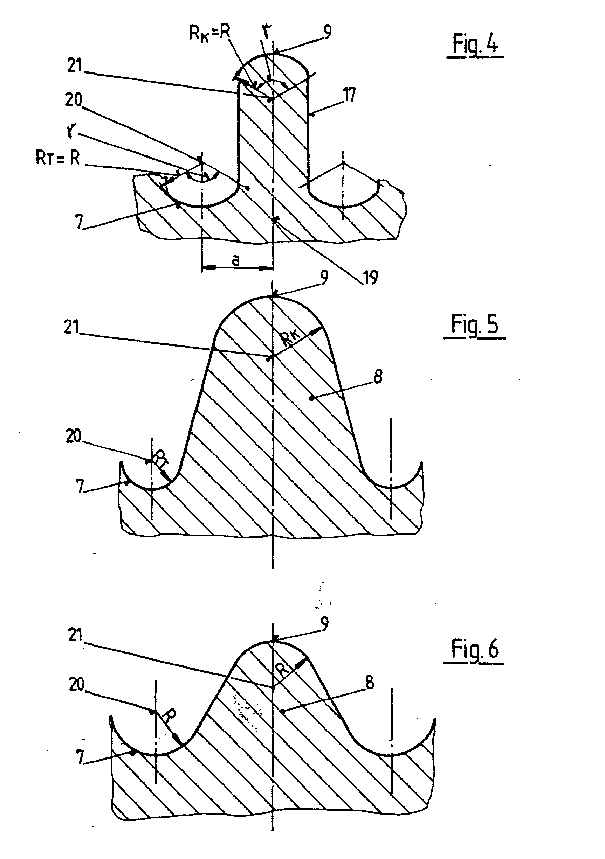

Die — bei eingesetzter Prothese mit ihren Flächennormalen nach anterior bzw. posterior weisenden — die Schmalseiten 2 und 5 verbindenden Blattseiten 6 des Schaftes stehen von distal nach proximal leicht konisch geöffnet zueinander (Fig. 2). Mit der Längsachse 1 bilden sie einen Oeffnungs-winkel α, der zwischen 1° und 10° beträgt. Dieser Winkel α ist gleichzeitig der Winkel, um den erfindungsgemäss die Talsohlen 7 (Fig. 4) einer rippenartigen Struktur im proximalen Höhenbereich H der Blattseiten 6 gegen die Längsachse 1 geneigt sind.

Bei der genannten Struktur erheben sich zwischen den dem Basisniveau der Blattseiten 6 entsprechenden Talsohlen 7 Rippen 8, die sich parallel zur Längsachse 1 des Schaftes erstrekken; die Scheitelhöhe (Fig. 3) der Rippenkämme 9 nimmt von distal nach proximal zu, wobei gegen die Längsachse 1 des Schaftes ein Oeffnungswinkel β (Fig. 3) entsteht, der erfindungsgemäss grösser als α ist; der Winkel β kann Werte zwischen 2° und 12° annehem.

Die neue Struktur ist nicht an einen Geradschaft gebunden; sie kann in gleicher Weise bei konventionellen Verankerungsschäften angewendet werden.

Wie bereits erwähnt, wird der Winkel β dabei so bemessen, dass auch die Rippenkämme 8 mindestens im wesentlichen noch von spongiosem Gewebe umgeben sind, da die gegenüber der Kortikalis relativ weiche Spongiosa als Dämpfung und Abfederung der Auflage wirkt.

Die Profilquerschnitte Rippenkämme 9 und der Talsohlen 7 werden aus stetig gekrümmten Kurven — im einfachsten Fall aus Kreisbögen mit Krümmungsradien von 1—6 mm gebildet. Der Aus Kurve ausgeschnittene Sektor 15 (Fig. 3) hat dabei einen Oeffnungswinkel γ von mindestens (2/3)·π, wobei mit Vorteil die zur Blattseite 6 vertikale Scheitelhöhe h so gross gewählt ist, dass die beiden Sektoren 15 bzw. 16 eines Rippenkammes 9 bzw. einer Talsohle 7 über eine ebene Fläche 17 miteinander verbunden sind.

Die Uebergänge 18 zwischen gekrümmten und ebenen Flächen werden dabei "sanft" ausgebildet, um Belastungsspitzen, die Ursachen für Knochenabbau und Ermüdungsbrüche sein können, zu vermeiden.

Ein Einwachsen von Knochengewebe in die Struktur lässt sich fördern, wenn die ebenen Flächen 17 als nach aussen gerichtete Flanken einer Rippe 8 zu der Mittelebene 19 (Fig. 4) durch einem Rippenkamm 9 höchstens parallel stehen oder damit einen spitzen Winkel bilden. Diese Forderung wird dadurch realisiert, dass parallel zur Blattseite oder Schaftoberfläche 6 der Abstand zweier benachbarter Krümmungsmittelpunkte 20 und 21 (Fig. 4) mindestens gleich der Summe der Krümmungsradien $R_K$ und $R_T$ der die Profilquerschnitte erzeugenden Kreise multipliziert mit dem Kosinus des Winkels (90—$\gamma$/2) ist; für $\gamma = \pi$ und gleiche Krümmungsradien $R_K = R_T = R$ ergibt sich daraus der einfache Spezialfall: a = 2·R, der jedoch nicht ausdrücklich dargestellt ist.

Die Schnitte der Fig. 5 und 6 zeigen eine weitere Variante für die erfindungsgemässe Rippenstruktur, bei der Talsohlen 7 und Rippenkämme 9 an ihrem proximalen Ende unterschiedliche Krümmungsradien $R_T$ und $R_K$ haben. Die relativ flache Krümmung mit dem Krümmungsradius $R_K$ der Rippenkämme 9 nimmt beim Fortschreiten vom proximalen Ende nach distal kontinuierlich bis auf einen Wert $R_K = R$ ab, während im Gegensinn dazu der Talsohlen-Krümmungsradius $R_T$ stetig bis zu dem gleichen Wert R anwächst, wie ein Vergleich der Fig. 5 und 6 zeigt.

Diese Ausbildung der Struktur mit variablen Krümmungsradien, deren Herstellung — wegen der komplizierteren Bearbeitung — aufwendiger ist als diejenige, bei der Talsohlen 7 und Rippenkämme 9 durchgehend einen konstanten Krümmungsradius in einem Profil haben — das z.B. mit Hilfe eines Formfräsers gefertigt werden kann —, ermöglicht eine besonders gute Verdichtung des spongiosen Knochengewebes beim Einschlagen der Prothese.

## Patentanspruche

1. Verankerungsschaft für eine Endoprothese, der in seinem proximalen Bereich mindestens auf Teilen seiner Oberfläche mit einer für das Einwachsen von Knochengewebe vorgesehen Oberflächenstruktur aus sich abwechselnden Rippen (8) und Tälern trägt, die in Richtung der Längsachse (1) des Verankerungsschaftes und gegen seine Längsachse (1) von distal nach proximal in einem spitzen Neigungswinkel verlaufen, dadurch gekennzeichnet, daß der Neigungswinkel $\alpha$ der zwischen den Rippen (8) gelegenen Talsohlen (7) gegen die Längsachse (1) $1° \leq \alpha \leq 10°$, vorzugsweise $2° \leq \alpha \leq 5°$, und der Neigungswinkel $\beta$ der Rippenkämme (9) größer als der zugehörige Neigungswinkel $\alpha$ ist, wobei der Neigungswinkel $\beta$ gleich $2° \leq \beta \leq 12°$, vorzugsweise $4° \leq \beta \leq 8°$ ist .

2. Verankerungsschaft nach Anspruch 1, dadurch gekennzeichnet, daß die Rippenkämme (9) und die Talsohlen (7) ein ovales, kreis- oder ellipsenförmiges Querschnittsprofil entsprechend einer stetig gekrümmten Kurve ohne Hinterschneidungen haben, wobei ferner der Öffnungswinkel $\gamma$ des abgerundeten Profilquerschnittsektors $\frac{2}{3}\pi \leq \gamma \leq \pi$ und der zur Schaftoberfläche (6) parallele Abstand (a) zweier benachbarter Krümmungsmittelpunkte eines Talsohlen- und eines Kammprofils mindestens gleich der Summe der Krümmungsradien der beiden Profile multipliziert mit cos (90°—$\gamma$/2 ist.

3. Verankerungsschaft nach Anspruch 2, dadurch gekennzeichnet, daß zum distalen Ende hin der Krümmungsradius der Talsohlen (7) zu- und derjenige der Rippenkämme (9) abnimmt.

## Revendications

1. Tige d'ancrage pour une endoprothèse, dont la région proximale présente, sur des parties au moins de sa surface de structure prévue pour l'enracinement du tissu osseux, des nervures et des creux alternés qui s'étendent en direction de l'axe longitudinal de la tige d'ancrage sous un angle aigu d'inclinaison de la zone distale à la zone proximale par rapport à cet axe, caractérisée en ce que l'angle $\alpha$ d'inclinaison des fonds (7) des creux situés entre les nervures (8), par rapport à l'axe longitudinal (1), vérifie la relation $1° \leq \alpha \leq 10°$, de préférence $2° \leq \alpha \leq 5°$, et l'angle $\beta$ d'inclinaison des arêtes (9) des nervures est plus grand que l'angle $\alpha$ correspondant, l'angle $\beta$ d'inclinaison vérifiant la relation $2° \leq \beta \leq 12°$, de préférence $4° \leq \beta \leq 8°$.

2. Tige d'ancrage selon la revendication 1, caractérisée en ce que les arêtes (9) des nervures et les fonds (7) des creux ont un profil de coupe transversale ovale, circulaire ou elliptique, correspondant à une ligne à courbure régulière sans contredépouilles, en outre l'angle $\gamma$ d'ouverture du secteur de coupe transversale du profil à arrondi vérifie la relation $2/3\pi \leq \gamma \leq \pi$ et la distance (a), parallèle à la surface de la tige (6), de deux centres de courbure voisins d'un profil de fond de creux de d'un profil d'arête est au moins égale à la somme des rayons de courbure des deux profils multipliée par cos (90°—$\gamma$/2).

3. Tige d'ancrage selon la revendication 2, caractérisée en ce que le rayon de courbure des fonds (7) des creux augmente et celui des arêtes (9) des nervures diminue en direction de l'éxtrémité distale.

## Claims

1. A fixing stem for an endoprosthesis, the stem having in its proximal zone over at least parts of its surface a surface structure adapted for invasion by bone tissue and comprising ribs (8) alternating with troughs which extend in the direction of and at an acute angle to the longitudinal axis (1) of the stem distally to proximally, characterised in that the inclination angle $\alpha$ to the longitudinal axis (1) of the trough bottoms (7) between the ribs (8) is $1° \leq \alpha \leq 10°$, preferably $2° \leq \alpha \leq 5°$, and the inclination angle $\beta$ of the rib combs (9) is greater than the associated inclination angle $\alpha$, the inclination angle $\beta$ being $2° \leq \beta \leq 12°$, preferably $4° \leq \beta \leq 8°$.

2. A fixing stem according to claim 1, characterised in that the rib combs (9) and the trough bottoms (7) have an oval or circular or elliptical cross-section shape corresponding to a continuous curve without reliefs, the opening angle $\gamma$ of the rounded profile cross-section sector being $2/3\pi \leq \gamma \leq \pi$ and the distance (a) parallel to the stem surface (6) between two adjacent curvature centres of a trough bottom cross-section and of a comb cross-section is at least equal to the sum of the radii of curvature of the two cross sections multiplied by cos(90°—$\gamma$/2).

2. A stem according to claim 2, characterised in that the radius of curvature of the trough bottoms (7) increases, and the radius of curvature of the rib combs (9) decreases, towards the distal end.

EP 0 141 022 B1

Fig.1

Fig.2

Fig.3

1

Fig. 4

$R_K = R$  9  17

21

20

$\gamma$

$R_T = R$

7

a

19

Fig. 5

9

21

$R_K$

8

20

$R_T$

7

Fig. 6

9

21

R

8

20

R

7